# EUROPEAN PATENT SPECIFICATION

(11) **EP 2 961 724 B1**
(45) Date of publication and mention of the grant of the patent: **16.08.2017**
(21) Application number: 14756969.3
(22) Date of filing: 25.02.2014
(51) Int. Cl.: C07C 51/265, C07C 63/04, C07C 63/26

(54) **PROCESS FOR OXIDATION OF CYMENES TO AROMATIC ACIDS**
VERFAHREN ZUR OXIDATION VON CYMOLEN ZU AROMATISCHEN SÄUREN
PROCÉDÉ D'OXYDATION DE CYMÈNES EN ACIDES AROMATIQUES

(30) Priority: 28.02.2013 US 201361770994 P
(43) Date of publication of application: 06.01.2016
(73) Proprietor: PERSTORP AB, 284 80 Perstorp (SE)
(72) Inventor: LUNDMARK, Stefan, 263 95 Farhult (SE); KANGAS, Matias, 252 20 Helsingborg (SE); HÄGGMAN, Bo, 224 65 Lund (SE)
(74) Representative: Hoffmann Eitle
(86) International application number: PCT/SE2014/000022
(87) International publication number: WO 2014/133433

(56) References cited:
- WO-A1-00/37406
- RU-C2- 2 415 836
- US-A1- 2010 168 461
- FANG RONG-QIAN ET AL.: 'Synthesis of p-isopropylbenzoic acid by catalytic oxidation of p-cymene' XIAMEN DAXUE XUEBAO, ZIRAN KEXUEBAN vol. 45, no. 6, 2006, pages 805 - 807, XP008180337
- J.L YNFIESTA ET AL.: 'Poliesters de acides bibasicos aromaticos. III. Oxidacion catalitica en fase vapor de p-Cimeno hasta acido tereftalico' ANALES DE LA REAL SOCIEDAD ESPAÑOLA DE FISICA Y QUIMICA vol. B49, 1953, pages 153 - 159, XP008180320
- D.V.N HARDY: 'Polyethylene Terephtalate and its early development' JOURNAL OF THE SOCIETY OF CHEMICAL INDUSTRY vol. 67, 1948, pages 426 - 428, XP055273997
- 'Gerlinde Lauterbach, Oxidation of Asymmetric p- dialkylbenzenes in the presence of various catalysts' JOURNAL FÜR PRAKTISCHE CHEMIE CHEMIKER-ZEITUNG vol. 336, 1994, pages 166 - 168, XP055274404

## Description

Disclosed is a process for production of aromatic carboxylic acids, in particular phthalic acids, such as terephthalic acid, isophthalic and/or o-phthalic acid or its anhydride, by a process comprising two or more steps including the steps of catalytic oxidation of a cymene, such as *p-*cymene, *m*-cymene and/or o-cymene, in gas phase, followed by a second oxidation in liquid phase, and crystallization using a plurality of series-connected crystallizers.

Until World War II, o-phthalic acid and later o-phthalic anhydride were manufactured primarily by liquid-phase oxidation of suitable feedstocks. The favored method was BASF's oxidation of naphthalene by sulphuric acid in the presence of mercury salts to form the anhydride. This process was patented in 1896. During World War I, a process to make *o-*phthalic anhydride by oxidation of naphthalene in the vapor phase over a vanadium and molybdenum oxide catalyst was developed in the United States. Air oxidation of 90% pure *o-*xylene to o-phthalic anhydride was commercialized in 1946. An advantage of o-xylene is the theoretical yield to o-phthalic anhydride of 1.395 kg/kg. With naphthalene, two of the ten carbon atoms are lost to carbon oxide formation and at most a 1.157 kg/kg yield is possible. Although both are suitable feedstocks, o-xylene is overwhelmingly favored.

Well in excess of 90% of the o-phthalic anhydride produced is obtained by oxidizing *o*-xylene in the vapor phase over a fixed bed catalyst. In the 1960s, there were two types of fixed-bed processes, low temperature/low space velocity and high temperature/high space velocity. Catalyst development resulted in higher allowable space velocities for the low temperature case while high yields were maintained. Consequently, use of the low temperature process which runs at <400°C has predominated. A commercially viable plant must operate at high selectivity of a least 75 mol% with a feed o-xylene concentration of 60 g/m³. This concentration is above the lower explosion limit of 43 g/m³. The catalyst should last at least three years. The multi-tube fixed-bed reactors typically have a 2.5 cm inside diameter tubes and heat from the highly exothermic oxidation reaction is removed by circulating molten salt. The catalyst combines two essential ingredients found in earlier catalysts, vanadium oxide and titanium dioxide, which are coated on an inert, non porous carrier in a layer typically being 0.02-2.0 mm thick.

A commercial plant which oxidized o-xylene in the liquid phase using acetic acid solvent and a cobalt/manganese/bromine catalyst system was operated in France starting about 1965 but was shut down in the late 1970s. The reaction produces o-phthalic acid, which is then dehydrated to the anhydride. Yields from this process are high; the yield to o-phthalic anhydride is at least 90 mol% vs. 80 mol% or less for vapor phase oxidation. Capital costs are high owing to the metallurgy required.

All technical grades of terephthalic acid is produced by catalytic, liquid-phase air oxidation of *p*-xylene. Several processes have been developed, but they all use acetic acid as a solvent and one or more multivalent heavy metal(s) as catalyst. Cobalt is always used. In the most popular process, cobalt and manganese are the multivalent heavy metal catalysts and bromine is the renewable source for free radicals. Acetic acid, air, *p*-xylene and catalyst(s) are fed continuously into an oxidation reactor that is maintained at 175-225°C and 1500-3000 kPa (1530 atm). Air is added in amounts in excess of stoichiometric requirements to minimize formation of byproducts. The oxidation is exothermic to the extent of 2 x 108 J/kg of *p*-xylene reacted and this heat is removed by allowing the acetic acid solvent to boil. The vapor is condensed and refluxed to the reactor and this sets the temperature/pressure ratio. The condensing vapor is used to generate steam, which is employed as a heat source in other part of the process. Two moles of water are formed per mole of *p*-xylene reacted. The residence time is typically between 30 min. and 2 hrs. depending on the process. More than 98% of the p-xylene is converted and the yield to terephthalic acid is at least 95 mol%. The effluent from the reactor is a slurry of terephthalic acid because it dissolves to a limited extent in almost all solvents, including the acetic acid/water solvent used here. This slurry passes through a surge vessel that operates at a lower pressure than the reactor. More terephthalic acid crystallizes and the slurry is then ready to be processed at close to atmospheric conditions. The terephthalic acid crystals are recovered by filtration, washed, dried, and conveyed to storage.

The oxidation of *p*-xylene is known to proceed through a free radical process. The Mn and Co metals alone are not strong enough oxidizers to start the radical chain reaction, instead it is initiated by forming bromine radicals from ions in solution. These bromine radicals then decompose hydroperoxides that are ligated to the metals as well as abstract hydrogens from the methyl groups on *p*-xylene to form free radicals and propagate the reaction. The radical chain reaction proceeds through a series of intermediates, starting with the oxidation of *p*-xylene to *p*-tolualdehyde, then *p*-toluic acid, 4-carboxybenzaldehyde, and finally to the terephthalic acid product.

Cymenes, such as the isomers o-cymene, *m*-cymene and *p*-cymene, are naturally occurring aromatic monoterpenoids insoluble in water, but miscible with ethanol and ether. *p*-Cymene is a constituent of a number of essential oils, most commonly the oil of cumin and thyme. During acid sulphite pulping and/or Kraft chemical wood pulping, α-pinene and some monocyclic monoterpenes are partially converted to cymenes. Crude cymenes can be separated from the digester gas relief condensates and purified by distillation. In the case of spruce and fir the distilled product is 99% *p*-cymene and only traces of other products, such as sesquiterpenoids, are present. Pine wood extractives contain 3-carene, which affords appreciable quantities *of m-*cymene besides *p*-cymene. After a said pulping of softwood, turpentine is recovered from the digester relief condensates. The sulphate turpentine composition and yield varies considerably depending on the wood species, growth conditions of the tree and storage time of the logs or chips. Typically, however the yield of crude turpentine after pulping is around 10 kg/ton of pulp. The crude turpentine is purified in the distillation process by which impurities, such as alkyl mercaptans and dialkyl sulphides as well as higher terpenoids are removed. At least half of the distilled sulfate turpentine is α-pinene and the next dominating component is 3-carene. The crude cymene can today be used within the mill as a resin-cleaning solvent and the distilled product finds use in the paint and varnish industry. Cymenes are intermediates in current commercial production of cresols from toluene. The alkylation of toluene with propene produces a mixture of cymene isomers. The mixture comprises, at the thermodynamic equilibrium at 200°C, typically ca. 5% o-cymene, ca. 65% *m*-cymene and ca. 30% *p*-cymene. Pd-catalyzed dehydrogenation of limonene ultimately leads to a mixture of intermediates including 10-15% of *p*-cymene. US 20100168461 A1 describes a two step process to prepare terephthalic acid from bio-based cymene. Both steps are carried out in the liquid phase. Terpenes are a renewable resource that may be used to replace present petroleum based sources of most of the industry's hydrocarbons. However, a turpentine or a mixture of terpenes, in and of itself, is not a commercially significant hydrocarbon feedstock. Therefore, a process that will readily convert a terpene or a turpentine feedstock into a valuable or commercially more acceptable compound is highly desirable.

It is an object of the present invention to provide a process wherein cymenes are oxidized to aromatic carboxylic acids, illustrated in Scheme 1 wherein *p*-cymene yields terephthalic acid. In a similar way, *m*-cymene can be converted to isophthalic acid and o-cymene to o-phthalic acid or its anhydride.

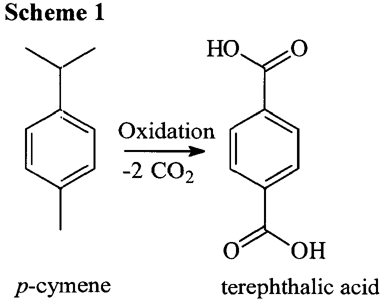

Further oxidations of cymenes to aromatic carboxylic acids, encompassed by the process of the present invention, include reactions and reaction products as illustrated by Schemes 2-4, wherein *p*-cymene yields various *p*-aromatic carboxylic acids. In similar ways, *m*-cymene can be converted to corresponding *m*-aromatic acids and o-cymene to corresponding o-aromatic acids or, where applicable, corresponding anhydride.

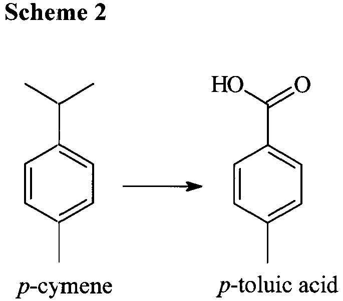

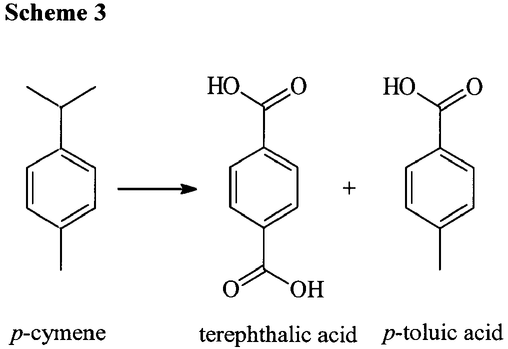

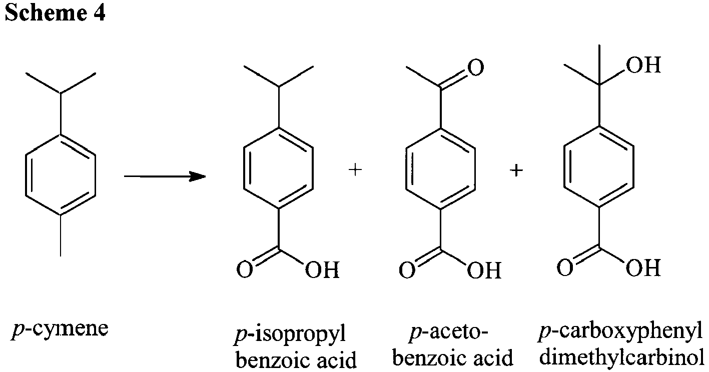

The oxidation of cymenes to aromatic acids, such as said phthalic acids, can be accomplished at elevated pressure using nitric acid, Mn(VII), permanganate, Mn(II), Co(II) or preferably in accordance with preferred embodiments of the present invention V₂O₅ or V₂O₅-TiO₂.

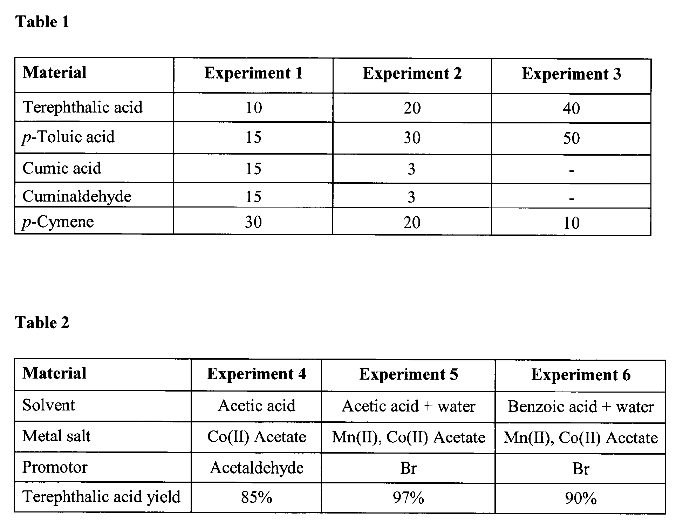

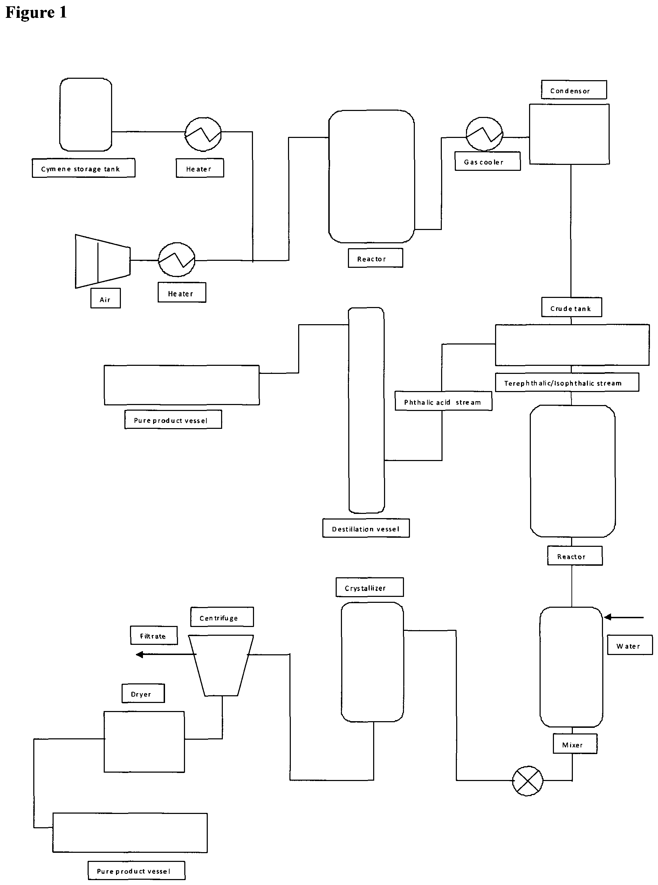

The overall process is described in enclosed Figure 1. Inert gases, excess oxygen, CO, CO₂ and volatile organic compounds (VOC) leave the gas/liquid separator and are sent to the high-pressure absorber. This stream is scrubbed with water under pressure, resulting in a gas stream of reduced VOC content. Part of the discharge from the high-pressure absorber is dried and is used as a source of inert gas, and the remainder is passed through a pressure control valve and a noise silencer before being discharged to the atmosphere through process vent (A). The underflow from the absorber is sent to the azeotrope still for recovery of acetic acid.

The reactor liquid containing a said aromatic, preferably a said phthalic, acid(s) flows to a series of crystallizers, where the pressure is relieved and the liquid is cooled by vaporization and return of condensed VOC and water. The partially oxidized impurities are more soluble in acetic acid and tend to remain in solution, while said aromatic acid(s) crystallize(s) from the liquor.

The slurry from the crystallizers is sent to solid/liquid separators, where a said acid, such as a said phthalic acid, is recovered as a wet cake. The wet cake from solid/liquid separation is sent to dryers, where with the use of heat, the moisture, predominately acetic acid, is removed leaving the product, as dry free flowing crystals.

In the presence of *p*-toluic acid, *p*-cymene is in the most preferred embodiments of the present process oxidized by air in two or more steps to terephthalic acid using V₂O₅, cobalt and manganese comprising catalysts. The first step performed in gas phase, and the second step in liquid phase. The reaction proceeds also in the absence of promoters or co-catalysts, such as acetaldehyde, paraldehyde, methyl ethyl ketone and/or bromine compounds and without solvents.

The selective oxidation of cymenes to the corresponding aromatic aldehydes and acids are effectively performed by vapor phase oxidation reaction over V₂O₅ or V₂O₅-TiO₂ combination catalyst with high activity and selectivity. Under appropriate conditions of oxidation of vapors of *o*-, *m-* and/or *p*-cymenes on V₂O_{5,} the main products are corresponding *o*-, *m-* or *p*-toluic acids, corresponding *o*-, *m-* or *p*-phthalic acids, carbon dioxide and water. In preferred embodiments of the present invention, cymenes are oxidized with an air flow of approximately 120-230 1/hr at 160-200°C for 1-3 hrs in the presence of a V₂O₅ comprising catalyst powder to give phthalic acids and toluic acids in approximately 40% and 50% yield respectively. MoO₂ and/or MoO₃ can also be used as catalyst, but V₂O₅ is the most preferred. The mechanism of the catalytic oxidation is the primary attack on the *iso*-propyl group, in contrast to the low temperature liquid phase oxidation where the products are cumic acid and cuminaldehyde. The pressures used in the present invention may be atmospheric or sub-atmospheric. Preferred sub-atmospheric pressures may range from about 100-200 mm Hg. The preferred catalytic amount of V₂O₅ used is 0.1-2.0% by weight of cymene to be oxidized

The reaction product from the first step gas phase oxidation, acetic acid, butyric acid and/or benzoic acid, water and a catalyst system, such as a manganese and/or cobalt acetate and sodium bromide system, are then combined into the liquid feed entering the reactor. The reactor type can for instance be bubble column, continuous tank reactor or loop-reactor. Air, compressed to a reaction pressure of about 2000 kPa (290 psi), is fed to the reactor. The temperature of the exothermic reaction is maintained at about 200°C by controlling the pressure at which the reaction mixture is permitted to boil and form the vapor stream leaving the reactor. A suitable ratio Co/Mn/Br are found within for instance 1/1-5/0.2-2.

Without further elaboration, it is believed that one skilled in the art can, using the preceding description, utilize the present invention to its fullest extent. The following preferred specific embodiments are therefore, to be constued as merely illustrative and not limitative. In the following, Examples 1 and 2 illustrate production of terephthalic acid from *p*-cymene, whereby Example 1 refer to step 1 gas phase oxidations and Example 2 to step 2 liquid phase oxidations. The result is given in enclosed Tables 1 and 2. Enclosed Figure 1 illustrate a suitable apparatus set up for production of aromatic carboxylic acids from cumens.

### Example 1 (Step 1 - gas phase oxidations)

Into a tubular reactor 2.5 cm in internal diameter and 3.5 m in length, the catalyst was packed together with an inert carrier. The tubular reactor was heated and a mixed gas containing *p-*cymene preheated to at least 135°C and air with an inlet temperature of 170°C, in a ratio of 100 g/Nm³ (100 gram of cymene relative to 1 m³ of air at standard temperature and pressure) was fed to into the tubular reactor at a rate of 4.0 Nm³/hr. Results are given in Table 1.

Three experiments (Experiment 1-3) were performed using the following reaction temperatures and reaction time:
Experiment 1: 160°C.
Experiment 2: 180°C.
Experiment 3: 200°C.
One hour reaction time.

Yielded reaction mixtures are in enclosed Table 1 reported as percent by weight.

### Example 2 (Step 2 - liquid phase oxidations)

A 11 stainless steel autoclave was charged with 500 grams of acidic solvent, 2 grams of Co/Mn/Br catalyst 1/1-5/0.2-2 molar ratio, a metal salt, a propmotor and 50 grams of the reaction product from the first oxidation step prepared according to Example 1. The reactants were heated to 180°C, and thereafter 4.0 Nm³/h of air were introduced into the liquid to a reaction pressure of ca. 290 psi. Upon completion of a two-hour reaction period, the autoclave was cooled and the crude product purified.

Three experiments (Experiment 4-6) were performed using the following reaction temperatures:
Experiment 4: 180°C.
Experiment 5: 200°C.
Experiment 6: 220°C.

Used solvents, metal salts and promotors as well as terephthalic acid yield in percent by weight are given in enclosed Table 2.

## Claims

1. A process in two or more steps for oxidation of a cymene to an aromatic carboxylic acid, wherein said process comprises a first oxidation step in gas phase with air or molecular oxygen in presence of a nitric acid, Mn(VII), permanganate, Mn(II), Co(II) and/or V₂O₅ comprising catalyst, followed by a second oxidation step in liquid phase in presence of a Co/Mn/Br catalyst.

2. A process according to claim 1, wherein said gas phase oxidation is performed in presence of a catalyst comprising V₂O_{5.}

3. A process according to claim 1 or 2, wherein a catalytic amount of V₂O₅ in a range of 0.1-1.0% by weight of cymene(s) are used.

4. A process according to any of the claims 1-3, wherein a ratio Co/Mn/Br of 1/1-5/0.2-2 is used.

5. A process according to any of the claims 1-4, wherein said cymene is o-cymene, *m-*cymene, *p*-cymene or a mixture thereof and said aromatic carboxylic acid is o-phthalic acid or its anhydride, isophthalic acid, terephthalic acid or a mixture thereof.

6. A process according to any of the claims 1-5, wherein said cymene are yielded from an α-pinene and/or a monocyclic monoterpene.

7. A process according to claim 6, wherein said α-pinene and/or said monocyclic monoterpene is yielded from acid sulphite pulping and/or Kraft chemical wood pulping.

8. A process according to claim 7, wherein said cymene is yielded from byproducts in a delignification process of acid sulphite pulping and/or Kraft chemical pulping.

9. A process according to any of the claims 1-8, wherein said process is performed at a reaction temperature ranging between 50°C and 350°C.

10. A process according to any of the claims 1-9, wherein said process is performed at a reaction temperature ranging between 100°C and 200°C.

11. A process according to any of the claims 1-10, wherein said cymene is o-cymene, *m-*cymene, *p*-cymene or a mixture thereof and said aromatic acid is o-phthalic acid or its anhydride, isophthalic acid, terephthalic acid or a mixture thereof.

## Patentansprüche

1. Verfahren in zwei oder mehr Schritten zur Oxidation eines Cymols zu einer aromatischen Carbonsäure, wobei das Verfahren einen ersten Oxidationsschritt in der Gasphase mit Luft oder molekularem Sauerstoff in Anwesenheit eines Salpetersäure, Mn(VII), Permanganat, Mn(II), Co(II) und/oder V₂O₅ umfassenden Katalysators, gefolgt von einem zweiten Oxidationsschritt in der Flüssigphase in Anwesenheit eines Co/Mn/Br-Katalysators umfasst.

2. Verfahren gemäß Anspruch 1, wobei die Gasphasenoxidation in Anwesenheit eines V₂O₅ umfassenden Katalysators durchgeführt wird.

3. Verfahren gemäß Anspruch 1 oder 2, wobei eine katalytische Menge an V₂O₅ in einem Bereich von 0,1-1,0 %, bezogen auf das Gewicht an Cymol(en) verwendet wird.

4. Verfahren gemäß einem der Ansprüche 1-3, wobei ein Co/Mn/Br-Verhältnis von 1/1-5/0,2-2 verwendet wird.

5. Verfahren gemäß einem der Ansprüche 1-4, wobei das Cymol o-Cymol, *m*-Cymol, *p*-Cymol oder eine Mischung davon ist und die aromatische Carbonsäure o-Phthalsäure oder ihr Anhydrid, Isophthalsäure, Terephthalsäure oder eine Mischung davon ist.

6. Verfahren gemäß einem der Ansprüche 1-5, wobei das Cymol aus einem α-Pinen und/oder einem monocyclischen Monoterpen gewonnen wird.

7. Verfahren gemäß Anspruch 6, wobei das α-Pinen und/oder das monocyclische Monoterpen aus saurem Sulfitaufschluss und/oder chemischem Kraft-Holzaufschluss gewonnen wird.

8. Verfahren gemäß Anspruch 7, wobei das Cymol aus Nebenprodukten bei einem sauren Sulfitaufschluss-Delignifizierungsverfahren und/oder einem chemischen Kraft-Aufschluss-Delignifizierungsverfahren gewonnen wird.

9. Verfahren gemäß einem der Ansprüche 1-8, wobei das Verfahren bei einer Reaktionstemperatur zwischen 50°C und 350°C durchgeführt wird.

10. Verfahren gemäß einem der Ansprüche 1-9, wobei das Verfahren bei einer Reaktionstemperatur zwischen 100°C und 200°C durchgeführt wird.

11. Verfahren gemäß einem der Ansprüche 1-10, wobei das Cymol o-Cymol, *m*-Cymol, *p*-Cymol oder eine Mischung davon ist und die aromatische Säure o-Phthalsäure oder ihr Anhydrid, Isophthalsäure, Terephthalsäure oder eine Mischung davon ist.

## Revendications

1. Processus en deux étapes ou plus pour l'oxydation d'un cymène en un acide carboxylique aromatique, dans lequel ledit processus comprend une première étape d'oxydation en phase gazeuse avec de l'air ou de l'oxygène moléculaire en présence d'un catalyseur comprenant de l'acide nitrique, du Mn (VII), du permanganate, du Mn (II), du Co (II) et/ou du V₂O₅, suivie par une seconde étape d'oxydation en phase liquide en présence d'un catalyseur au Co/Mn/Br.

2. Processus selon la revendication 1, dans lequel ladite oxydation en phase gazeuse est effectuée en présence d'un catalyseur comprenant du V₂O₅.

3. Processus selon la revendication 1 ou 2, dans lequel on utilise une quantité catalytique de V₂O₅ dans une plage de 0,1 à 1,0 % en poids de cymène(s).

4. Processus selon l'une quelconque des revendications 1 à 3, dans lequel on utilise un rapport Co/Mn/Br de 1/1-5/0,2-2.

5. Processus selon l'une quelconque des revendications 1 à 4, dans lequel ledit cymène est du o-cymène, du *m*-cymène, du *p*-cymène ou un mélange de ceux-ci et ledit acide carboxylique aromatique est de l'acide *o*-phtalique ou son anhydride, de l'acide isophtalique, de l'acide téréphtalique ou un mélange de ceux-ci.

6. Processus selon l'une quelconque des revendications 1 à 5, dans lequel ledit cymène est produit à partir d'un α-pinène et/ou d'un monoterpène monocyclique.

7. Processus selon la revendication 6, dans lequel ledit α-pinène et/ou ledit monoterpène monocyclique est produit à partir de la fabrication de pâte de sulfite acide et/ou de pâte de bois chimique Kraft.

8. Processus selon la revendication 7, dans lequel ledit cymène est produit à partir de sous-produits dans un processus de délignification de fabrication de pâte de sulfite acide et/ou de pâte chimique Kraft.

9. Processus selon l'une quelconque des revendications 1 à 8, dans lequel ledit processus est réalisé à une température de réaction variant entre 50°C et 350°C.

10. Processus selon l'une quelconque des revendications 1 à 9, dans lequel ledit processus est réalisé à une température de réaction variant entre 100°C et 200°C.

11. Processus selon l'une quelconque des revendications 1 à 10, dans lequel ledit cymène est du o-cymène, du *m*-cymène, du p-cymène ou un mélange de ceux-ci et ledit acide aromatique est de l'acide o-phtalique ou son anhydride, de l'acide isophtalique, de l'acide téréphtalique ou un mélange de ceux-ci.
